(19) Europäisches Patentamt
European Patent Office
Office européen des brevets

(11) Publication number: **0 410 446 A1**

# (12) EUROPEAN PATENT APPLICATION

(21) Application number: **90114356.0**

(22) Date of filing: **26.07.90**

(51) Int. Cl.⁵: **C07C 59/01**, C07C 51/09, A61K 31/19

(30) Priority: **26.07.89 JP 193478/89**

(43) Date of publication of application: **30.01.91 Bulletin 91/05**

(84) Designated Contracting States: **AT BE CH DE DK ES FR GB GR IT LI LU NL SE**

(71) Applicant: **MOCHIDA PHARMACEUTICAL CO., LTD.**
**7, Yotsuya 1-chome**
**Shinjuku-ku Tokyo 160(JP)**

(72) Inventor: **Murakami, Kimihiro, c/o Mochida Pharmaceutical Co.**
**Ltd., 7, Yotsuya 1-chome, Shinjuku-ku**
**Tokyo 160(JP)**
Inventor: **Niho, Takeshi, c/o Mochida Pharmaceutical Co.**
**Ltd., 7, Yotsuya 1-chome, Shinjuku-ku**
**Tokyo 160(JP)**
Inventor: **Ishikawa, Hiroshi, c/o Mochida Pharmaceutical Co.**
**Ltd., 7, Yotsuya 1-chome, Shinjuku-ku**
**Tokyo 160(JP)**
Inventor: **Mochida, Ei, c/o Mochida Pharmaceutical Co.**
**Ltd., 7, Yotsuya 1-chome, Shinjuku-ku**
**Tokyo 160(JP)**

(74) Representative: **Hansen, Bernd, Dr. Dipl.-Chem. et al**
**Hoffmann, Eitle & Partner Patent- und Rechtsanwälte Arabellastrasse 4e 4**
**D-8000 München 81(DE)**

## (54) (S)-5-hydroxydecanoic acid and its therapeutic composition.

(57) The present invention relates to a compound selected from the group consisting of (S)-5-hydroxydecanoic acid, a salt, a solvate and a solvate of said salt thereof and relates to methods for producing the compound, comprising of inversion of the configuration and also relates to a pharmaceutical composition for treatment of cardiovascular diseases such as arrhythmia and ischemic heart diseases, or diabates.

EP 0 410 446 A1

## (S)-5-HYDROXYDECANOIC ACID AND ITS THERAPEUTIC COMPOSITION

### Background of the invention

The present invention relates to a compound selected from the group consisting of novel (S)-5-hydroxydecanoic acid ((S)-5-HDA) represented by the formula (I):

$$CH_3\text{-}(CH_2)_4\text{-}\underset{(S)}{\overset{OH}{\overset{|}{C}}}H\text{-}(CH_2)_3\text{-}CO_2H \qquad (I),$$

a pharmaceutically acceptable salt, a solvate and a solvate of said salt thereof, which is useful for treatment of cardiovascular diseases or diabetes and relates to a method for producing the compound and also relates to a pharmaceutical composition comprising the compound as a pharmaceutically active component.

It is known that racemic 5-hydroxydecanoic acid (5-HDA) represented by the formula (II):

$$CH_3\text{-}(CH_2)_4\text{-}\overset{OH}{\overset{|}{C}}H\text{-}(CH_2)_3\text{-}CO_2H \qquad (II),$$

its salt and/or its ester has an excellent therapeutic effect on hyperlipidemia, a preventing effect on impairment of mitochondorial functions in a model due to ischemia, and a notable anti-arrhythmic effect without cardiodepressant effects. And 5-HDA is very safe with little toxicity (Ohnishi, H., et al ., Jpn. Kokai Tokkyo Koho JP 59-1,415). Racemic sodium 5-hydroxydecanoate also has an excellent therapeutic effect on model of ischemic arrhythmia and one of its mechanisms of action is the inhibition of adenosine triphosphate (ATP) regulated potassium ($K^+$) channels at myocardium (Niho, T., et al ., Folia Pharmacol. Japon., 89 155-167, 1987). However, 5-HDA, its salt or its ester has a problem of difficulty to be made into composition and a problem of distress on patients in administration, because its therapeutically effective doses are generally high. Therefore, a therapeutic agent which is stronger pharmacologically and therapeutically effective and is more useful at lower dose than 5-HDA derivative is required.

On the other hand, a compound of the present invention, i.e. (S)-5-HDA, a pharmaceutically acceptable salt, a solvate or a solvate of said salt thereof, has never been isolated nor produced yet. And physicochemical and pharmacological properties of this optically active compound are further not known.

As for an optically active δ-decalactone, which is a starting compound of the present invention, both of its (R)-isomer and (S)-isomer are known compound (Utaka, M., et al ., J. Organic Chemistry, 52 4363-4368, 1987; Jpn. Kokai Tokkyo Koho JP 61-191,679; Tuynenburg Muys, G., et al ., Applied Microbiol., 11 389-393, 1963; Nishida, F., et al ., Proceeding of the 29th symposium on the chemistry of natural products, 729-735, 1987; Kohver, O., Tetrahedron, 26 2391-2396, 1970; Blanco, L., et al ., Tetrahedron Lett., 29 1915-1918, 1988; and Mosandl, A., et al ., Z. Lebensm. Unters. Forsch., 187 40-44, 1988).

(R)-δ-decalactone can be readily prepared in good optical purity by reduction of 5-oxodecanoic acid with baker's yeast, according to the methods described by Utaka, M., et al ., (J. Organic Chemistry, 52 4363-4368, 1987) and by Tuynenburg Muys, G., et al ., (Applied Microbiol., 11 389-393, 1963). On the other hand, only a few examples of preparation of (S)-δ-decalactone are described in proceeding papers (Tuynenburg Muys, G., et al ., Applied Microbiol., 11 389-393, 1963; Blanco, L., et al ., Tetrahedron Lett., 29 1915-1918, 1988; Jpn. Kokai Tokkyo Koho JP 61-191,679; and Mosandl, A., et al ., Z. Lebensm. Unters. Forsch., 187 40-44, 1988). The method described by Tuynenburg Muys, G., et al ., comprising reduction of 5-oxodecanoic acid with a filamentous fungus ( Cladosporium ) is not carried out practically, because Cladosporium is generally difficult to purchase and an optical purity of resulting (S)-δ-decalactone is low.

The method described by Blanco, L., et al ., that (S)-δ-decalactone can be prepared from racemic δ-decalactone by treatment with a lipase, is not carried out industrially, because a lipase is unstable and an optical purity of the product is low. The method described in Jpn. Kokai Tokkyo Koho JP 61-191,679 is an optical resolution of racemic δ-decalactone by HPLC using a method of chiral column chromatography. And the method by Mosandl, A., et al ., is that racemic δ-decalactone mixture is converted into a mixture of diastereomers by reacting with an optically active acid-chloride, then each diastereomers are resoluted by gas- or liquid- chromatography. But, any resolution method above is very difficult to carry out on an industrial scale. Therefore there is no easy method for producing (S)-δ-decalactone which is a starting compound of the present invention inexpensively and industrially, so it has been desired to develop a simple method to carry out.

## Detailed Description of The Invention

As a result of an extensive investigation concerning optically active derivatives of the 5-HDA for noticing its asymmetric carbon atom, the present inventors have found that: a) (S)-δ-decalactone which is very difficult to obtain can be prepared at good optical purity from easily purchased (R)-δ-decalactone by following method. (R)-δ-decalactone is hydrolyzed and the carboxyl group of the product is protected by esterification. Then, the esterified compound is converted to the (S)-acyl derivative, comprising inversion of the configuration. The resulting (S)-acyl derivative is hydrolyzed of its ester groups and is cyclized in acidic condition to be (S)-δ-decalactone. In the present invention, said acyl derivative means a compound acylated at 5-position of 5-HDA derivative; b) the compound of the present invention such as (S)-5-HDA and its salt can be prepared at good optical purity by hydrolysis of the above obtained (S)-δ-dacalactone; c) the compound of the present invention can be also prepared without through (S)-δ-decalactone by following method. (R)-δ-decalactone is hydrolyzed and the carboxyl group of the product is protected by esterification. Then the esterified compound is converted to the (S)-acyl derivative, comprising inversion of the configuration and the resulting (S)-acyl derivative is hydrolyzed; and d) (S)-δ-dacalactone has a notable pharmacological effect as compared with racemic mixture.

A compound of the present invention represented by the formula (I) may be converted into pharmacologically acceptable salts with inorganic or organic bases. Suitable salts are, for example, salts with alkali metal such as lithium, sodium and potassium; salts with alkaline-earth metal such as calcium and barium; salts with inorganic bases such as ammonium; salts with amino acids such as lysine and arginine; and salts with organic bases such as benzylamine, diethylamine, pyridine, ethanolamine, procaine, choline and N-methylglucosamine.

A compound of the present invention represented by the formula (I) and its salt may be converted into pharmaceutically acceptable solvates such as hydrate.

A compound of the present invention of formula (I) can be generally prepared as follows. A known (R)-δ-decalactone is hydrolyzed with an alkali and the carboxyl group of the product is protected by esterification. Then, the esterified compound is converted to the (S)-acyl derivative, comprising inversion of the configuration at 5-position by reacting with an organic carboxylic acid in the presence of an azodicarboxylic acid dialkyl ester and triphenylphosphine. The resulting (S)-acyl derivative is hydrolyzed of its ester group and the resulting compound is cyclized in acidic condition to be (S)-δ-decalactone. After hydrolysis of (S)-δ-decalactone with an alkali such as sodium hydroxide, potassium hydroxide, lithium hydroxide, calcium hydroxide, or barium hydroxide, free (S)-5-HDA can be prepared by adjusting the pH to 2-3 approximately with a mineral acid such as hydrochloric acid or sulfuric acid, or an organic acid such as citric acid. The resulting free (S)-5-HDA can be treated with one equivalent of a base to produce a salt of (S)-5-HDA. Suitable salts are salts with alkali metals such as litium, sodium and potassium; salts with alkaline earth metals such as calcium and barium; salts with inorganic bases such as ammonium; salts with amino acids such as lysine and arginine; and salts with organic bases such as benzylamine, diethylamine, pyridine, ethanolamine, procaine, choline and N-methylglucosamine. All reactions of hydrolysis in the present invention can be carried out in water or a mixed solvent of water and organic solvent such as methanol, ethanol, or acetone at 0° C to 100° C, preferably at ambient temperature to 80° C.

A salt of compound of the present invention can be also prepared by hydrolysis of (S)-δ-decalactone with one equivalent of an alkali such as sodium hydroxide, potassium hydroxide, lithium hydroxide, calcium hydroxide or barium hydroxide, in water or a mixed solvent of water and organic solvent such as methanol, ethanol or acetone and then by lyophilization or other drying methods.

Further a compound of the present invention can be prepared, without through (S)-δ-decalactone, by

hydrolysis of (S)-acyl derivative of 5-HDA, which has been prepared by above reaction of inversion of the configuration of (R)-acyl derivative of 5-HDA, with 2 to 10 equivalents of alkali such as sodium hydroxide, potassium hydroxide, lithium hydroxide, calcium hydroxide or barium hydroxide, in water or a mixed solvent of water and organic solvent such as methanol, ethanol or acetone. Then the hydrolyzed mixture can be purified by column chromatography such as Daiaion HP-20® (Mitsubishi Chemical Industries LTD, Japan), Daiaion CHP-20P® (Mitsubishi Chemical Industries LTD, Japan) or Amberlite XAD-2® (Rohm & Hass, USA).

The method for producing (S)-δ-decalactone from (R)-δ-decalactone with inversion of the configuration is described in detail as follows.

An inversion of the configuration of δ-decalactone can be generally carried out by the following three processes.

The first process (esterification)

After hydrolysis of (R)-δ-decalactone with an alkali such as sodium hydroxide or potassium hydroxide, free 5-HDA is obtained from the reaction mixture by extraction in acidic condition, and then a carboxyl group of 5-HDA is esterified by usual methods. As the esters used in this process, alkyl esters such as methyl ester or ethyl ester, or aralkyl esters such as optionally substituted benzyl ester or diphenylmethyl ester are included. The configuration at 5-position of the substances are retained completely without racemization throughout this process.

The second process (acylation with an inversion of the configuration)

The esterified compound of (R)-configuration at 5-position prepared in the first process, can be converted to the (S)-acyl derivative with an organic carboxylic acid in the presence of an azodicarboxylic acid dialkyl ester and triphenylphosphine. Suitable solvents in this process include organic solvents such as ether, dioxane, tetrahydrofuran, chloroform, methylene chloride or benzene, and the reaction is carried out at -50 °C to 50 °C, preferably at 0 °C to ambient temperature. Organic acids usable in this process are benzoic acid, acetic acid, propionic acid, phenylacetic acid or substituted benzoic acid, and so on.

The third process (deprotection, lactonization)

After hydrolysis of the (S)-acyl derivative prepared in the second process with an alkali such as sodium hydroxide or potassium hydroxide, free (S)-5-HDA is obtained from the reaction mixture by extraction and is reacted with an acid to obtain (S)-δ-decalactone. Any organic or inorganic acid can be used to cyclize free (S)-5-HDA in this process. In this process, after hydrolysis of ester, above mentioned lactonization can be proceeded by addition of an excess amount of acid to the reacting mixture without isolation of free (S)-5-HDA.

In any case of this process, the configuration at 5-position is retained completely without racemization. The δ-decalactone obtained in this process has (S)-configuration at 5-position which is opposite to the configuration of δ-decalactone used as a starting compound in the first process, because in the second process, the configuration of an acyl derivative has been inverted completely.

The efficacy, safety, usage and dosage of (S)-5-HDA and its salts and the methods for producing pharmaceutical preparation containing one of them are described in the following experiments and examples.

Experiment 1 Effect on ATP regulated $K^+$ channels in guinea-pig cardiac cells

According to the method described by Kakei, M., et al ., ( J. Physiol., 363 441-462, 1985 ), single cardiac cells were prepared. Guinea-pigs were anesthetized with an intraperitoneally injection of pentobarbital sodium with 30 mg/kg and the chest was opened under artificial respiration. The aorta was cannulated in situ to perfuse the coronary arteries with Tyrode solution (NaCl 136.1, KCl 5.4, $CaCl_2$ 1.8, $MgCl_2$ 0.5, $NaH_2PO_4$ 0.33, glucose 5.5, N-2-hydroxyethylpiperazine-N′-2-ethanesulfonic acid (HEPES) 5.0, each in mM, and the pH was adjusted to 7.4 with NaOH), then the heart was dissected out and mounted on Langendorff apparatus to perfuse. After blood was washed out by Tyrode solution, the heart was perfused with the 100

ml of $Ca^{2+}$-free Tyrode solution and then with $Ca^{2+}$-free Tyrode solution containing 0.17 mg/ml collagenase for 5-20 minutes. Subsequently, the enzyme was washed out from the heart with the 100 ml of KB solution (taurine 10, oxalic acid 10, glutamic acid 80, KCl 25, $KH_2PO_4$ 10, HEPES 10, glucose 11, ethyleneglycolbis-($\beta$-aminoethylether)N,N′-tetraacetic acid (EGTA) 0.5, each in mM, and the pH was adjusted to 7.4 with KOH) and the heart was stored in KB solution at 4°C for at least 1 hour. Before electrical recording, a part of ventricular muscle was dissected and was shaken in recording chamber (0.7 ml) filled with Tyrode solution to disperse each cells. The method of single channel electrical recording was essentially the same as described by Hamill, A., et al ., (Pflugers Arch., 391 85-100, 1981 ). Glass electrodes were made from Pyrex glass capillaries by use of a puller (2 - 10 MΩ) and were heated to polish just before use. An electrode solution was KCl 5.4, NaCl 144.6, $CaCl_2$ 2, HEPES 5 (each in mM) and the pH was adjusted to 7.4 with NaOH. After dispersed cells sank and adhered to the bottom of recording chamber, high $K^+$ solution (KCl 140, $MgCl_2$ 1.0, $KH_2PO_4$ 0.5, EGTA 1.0, HEPES 5.0, each in mM, and the pH was adjusted to 7.4 with KOH) was perfused at flow rate of 2 - 3 ml/minute. After established a high seal resistance (5 - 20 GΩ) obtained by attaching an electrode to a cell membrane and applying suction (0 - -50 cm $H_2O$), a channel current was detected by inside-out configuration by use of a patch clamp amplifier (8900, DAGAN) at a filter setting of 1 KHz. A channel current and a membrane potential were monitored by an oscilloscope and were recorded by a datarecorder. An opening probability, that is a ratio of channel opening period to recording period, were analyzed by using a p-CLAMP (AXON) at 0 mV of a membrane potential. Compounds were dissolved in high $K^+$ solution to be perfused. The result is shown in table 1.

Table 1

| The inhibitory effect of optical isomers of sodium 5-hydroxydecanoate on ATP regulated $K^+$ channels | | | |
|---|---|---|---|
| | Dose (M) | Inhibition rate (%) | N |
| (R,S)-5-HD | $3x10^{-4}$ | 30 | 4 |
| (R)-5-HD | $3x10^{-4}$ | 10 | 7 |
| (S)-5-HD | $3x10^{-4}$ | 69 | 9 |
| 5-HD; Sodium 5-hydroxydecanoate | | | |

The above result shows that (S)-isomer has an inhibitory effect on ATP regulated $K^+$ channels about twice as good as racemate and (R)-isomer has few activity.

Experiment 2 Acute toxicity

Five male ddY mice weighting about 25 g were orally administered once or intraperitoneally injected once with 1 g/kg of sodium (S)-5-hydroxydecanoate. The mice were observed for one week, and no abnormality occurred and no animal died.

From the above results, it is exceedingly desired that (S)-5-HLA, as a compound of the present invention, may have therapeutic effects on arrhythmia and/or ischemic heart diseases, one of these etiologic mechanisms may be related to activation of ATP regulated $K^+$ channels, because (S)-5-HDA of the present invention has an inhibitory effect on that channel.

It is also suggested that ATP regulated $K^+$ channels exist in pancreatic $\beta$-cells and are involved to insulin secretion. Practically, tolbutamide, which is used as therapeutic composition for diabates, has an accelerative effect on insulin secretion based on its obvious inhibitory effect on ATP regulated $K^+$ channels Nicholas, C., et al ., Lancet, 2 8453 474-475, 1985). Therefore, it is suggested that a compound of the present invention, which inhibits ATP regulated $K^+$ channels, may be useful as therapeutic composition for diabetes, too.

Furthermore, a compound of the present invention has many pharmacological effects such as an anti-arrhythmic effect and a protective effect on ischemic heart in experimental animal models and has large margin for safety at higher doses than its pharmaceutically effective one. A compound of the present

invention, therefore, is very useful and is desired to be a therapeutic and/or preventive composition for cardiovascular diseases such as arrhythmia and ischemic heart diseases.

A pharmaceutical composition of the present invention is used in an amount of 2.5 to 10,000 mg/day for an adult as (S)-5-HDA, when it is used as therapeutic and/or preventive composition for cardiovascular diseases or diabetes. For oral administration, for intrarectal administration and for injection, the preferred doses are from 50 to 5,000 mg, from 50 to 500 mg and from 5 to 5,000 mg, respectively. The exact dose may vary according to the route of administration and the severity of the disease. It can be administrated at a time or divided several times or an intravenous drip.

Pharmaceutical preparations can be produced from the pharmaceutical composition of the present invention by any conventional technique using any conventional pharmaceutical carrier, vehicle or excipient.

Pharmaceutical preparations include oral preparations such as capsules, tablets, granules, powders and oral liquid formulations, and rectal preparations such as rectal suppositories. As injectable forms, emulsions for injection, aqueous solutions for injection and lyophilized solids which are dissolved just before use, are included.

Details of the present invention are described in the following examples. The present invention is not limited within the following examples.

The measuring method of an optical purities described in examples was carried out as the following. Optically active 5-HDA and its salts were esterified with diazomethane and the resulting compounds were converted into carbamate derivatives by reaction with 3,5-dinitrophenylisocyanate. Optical purities of an optically active 5-HDA and its salts were determined as the optical purities of the above carbamate derivatives, measuring by a HPLC (Eluate, hexane:1,2-dichloroethane:ethanol = 100:20:1) using a column contained optically active solid support (SUMIPAX OA-2100I®).

Example 1

Preparation of (S)-δ-decalactone.

Step 1

Preparation of (R)-δ-decalactone.

To a solution of potassium dihydrogen phosphate (1.35 g), magnesium sulfate (0.77 g) and glucose (670 g) in water (1.7 L) was added baker's yeast (600 g) at 30 °C. After stirring at 28 - 30 °C for 30 minutes, a solution of 5-oxodecanoic acid (20.0 g) in 0.1 M potassium hydroxide solution (1 L) and glucose (300 g) were added to the suspension, and the pH of the suspension was adjusted to 6 - 7 with 1 M potassium hydroxide solution. The resulting suspension was stirred for 3 hours and allowed to stand for 14 hours at 28 - 30 °C. Celite® (300 g) was added and the mixture was stirred for 2 hours. After filtering off the insoluble matters, the filtrate was acidified with 1 M hydrochloric acid to pH 2 and extracted twice with ether (3 L). The extract was washed with brine (2 L), dried over anhydrous sodium sulfate and concentrated under reduced pressure. The residue was dissolved in benzene (150 ml), and the solution was refluxed with p-toluenesulfonic acid monohydrate (0.86 g) for 30 minutes. After cooling to ambient temperature, the reaction mixture was washed successively with brine (50 ml), saturated sodium bicarbonate solution (50 ml), and brine (50 ml). The organic layer was dried over anhydrous sodium sulfate and concentrated under reduced pressure. The residue was purified by silica gel column chromatography (acetone:hexane = 1:7) to give 8.2 g of the objective compound.

$[\alpha]_D^{30}$ = +58.2° (c = 1.758, tetrahydrofuran)
IR (KBr, $cm^{-1}$): 2931, 2862, 1735, 1245, 1052, 1035
NMR ($CDCl_3$, δ): 4.40 - 4.00 (1H, m), 2.64 - 2.28 (2H, m), 2.07 - 1.02 (12H, m), 0.89 (3H, t)

Step 2

Preparation of methyl (R)-5-hydroxydecanoate.

A mixture of the product obtained in Step 1 (6.9 g) and 1 M sodium hydroxide solution (38.2 ml) was stirred at 50 - 60 °C for 30 minutes. After cooling to ambient temperature, the resulting solution was acidified under ice-cooling with 1 M hydrochloric acid to pH 2, and extracted twice with ether (50 ml). The extract was washed with brine (50 ml), dried over anhydrous sodium sulfate, and concentrated to 40 ml under reduced pressure. After methylation with ethereal diazomethane, the solvent was removed under reduced pressure to give 7.7 g of the objective compound.
NMR ($CDCl_3$, δ): 3.84 - 3.52 (1H, m), 3.67 (3H, s), 2.35 (2H, t), 2.00 - 1.20 (12H, m), 0.89 (3H, t)

Step 3

Preparation of methyl (S)-5-benzoyloxydecanoate.

To an ice-cooled solution of diethyl azodicarboxylate (6.62 g) and benzoic acid (4.64 g) in ether (60 ml) was added dropwise a solution of the product obtained in Step 2 (6.40 g) and triphenylphosphine (9.97 g) in ether (60 ml) over a 8-minute period, and the mixture was stirred for 1 hour. After filtering off the insoluble matters, the filtrate was concentrated under reduced pressure. The residue was purified by silica gel column chromatography (acetone:hexane = 2:98 - 5:95) to give 7.38 g of the objective compound.
IR (neat, $cm^{-1}$): 2955, 1735, 1718, 1272, 1114, 713
NMR ($CDCl_3$, δ): 8.06 (2H, dd), 7.57 - 7.36 (3H, m), 5.25 - 5.04 (1H, m), 3.66 (3H, s), 2.50 - 2.25 (2H, m), 1.90 -1.50 (6H, m), 1.50 - 1.08 (6H, m), 0.87 (3H, t)

Step 4

Preparation of (S)-δ-decalactone.

To a solution of sodium hydroxide (0.67 g) in water (17 ml) and methanol (50 ml) was added the product obtained in Step 3 (0.85 g), and the mixture was refluxed for 2 hours. After cooling to ambient temperature, methanol was evaporated under reduced pressure and water (50 ml) was added to the residue. The pH of the solution was adjusted to about 2 with 1 M hydrochloric acid, and the acidified mixture was extracted twice with ethyl acetate (50 ml). The extract was washed with brine (50 ml), dried over anhydrous sodium sulfate and concentrated under reduced pressure. The residue was dissolved in benzene (50 ml), and the solution was refluxed with p-toluenesulfonic acid monohydrate (10 mg) for 10 minutes. After cooling to ambient temperature, the reaction mixture was washed successively with brine (30 ml), saturated sodium bicarbonate solution (30 ml), and brine (30 ml). The organic layer was dried over anhydrous sodium sulfate and concentrated under reduced pressure. The residue was purified by silica gel column chromatography (acetone:hexane = 1:4) to give 0.41 g of the objective compound.
$[\alpha]_D^{30}$ = -57.9° (c = 1.783, tetrahydrofuran)
IR (KBr, $cm^{-1}$): 2931, 2862, 1735, 1245, 1052, 1035
NMR ($CDCl_3$, δ): 4.40 - 4.00 (1H, m), 2.64 - 2.28 (2H, m), 2.07 - 1.02 (12H, m), 0.89 (3H, t)

Example 2

Preparation of sodium (S)-5-hydroxydecanoate.

A mixture of (S)-δ-decalactone obtained in Example 1 (1.0 g) and 0.5 M sodium hydroxide solution (11.76 ml) was stirred at 50 - 60 °C for 20 minutes. After cooling to ambient temperature, the reaction mixture was lyophilized to give 1.21 g of the objective compound.
melting point: 161.0 - 162.4 °C
$[\alpha]_D^{26}$ = -2.3° (c = 1.446, methanol)
optical purity (HPLC): >98% e.e.
IR (KBr, $cm^{-1}$) : 2964, 2936, 1636, 1578, 1396
NMR (DMSO-$d_6$, δ): 3.60 - 3.12 (1H, m), 1.94 - 1.74 (2H, m), 1.74 - 1.02 (12H, m), 0.86 (3H, t)

Example 3

Preparation of sodium (S)-5-hydroxydecanoate.

To a solution of sodium hydroxide (0.24 g) in water (7 ml) and methanol (20 ml) was added the product obtained in Step 3 of Example 1 (306 mg) and the mixture was refluxed for 2 hours. After cooling to ambient temperature, methanol was evaporated under reduced pressure and the pH of the aqueous solution was adjusted to about 8 with 1 M hydrochloric acid. The resulting solution was applied to a column of Diaion HP-20®. The objective fractions eluted with water were collected and lyophilized to give 162 mg of the objective compound.
melting point: 161.0 - 162.4 °C
$[\alpha]_D^{25}$ = -2.3 ° (c = 1.435, methanol)
optical purity (HPLC): >98% e.e.
IR (KBr, $cm^{-1}$): 2964, 2936, 1636, 1578, 1396
NMR (DMSO-$d_6$, δ): 3.60 - 3.12 (1H, m), 1.94 - 1.74 (2H, m), 1.74 - 1.02 (12H, m), 0.86 (3H, t)

Example 4

Preparation of potassium (S)-5-hydroxydecanoate.

A mixture of (S)-δ-decalactone obtained in Example 1 (1.23 g) and 0.5 M potassium hydroxide solution (14.5 ml) was stirred at 50 - 60 °C for an hour. After cooling to ambient temperature, the reaction mixture was washed with ether (5 ml) and lyophilized to give 1.67 g of the objective compound.
melting point: 169.7 - 170.9 °C
$[\alpha]_D^{25}$ = -1.9 ° (c = 1.428, methanol)
optical purity (HPLC): >98% e.e.
IR (KBr, $cm^{-1}$): 2958, 2928, 1654, 1559, 1407
NMR (DMSO-$d_6$, δ): 3.54 - 3.30 (1H, m), 1.98 - 1.68 (2H, m), 1.62 - 1.02 (12H, m), 0.85 (3H, t)

Example 5

Preparation of (S)-5-hydroxydecanoic acid.

A mixture of (S)-δ-decalactone obtained in Example 1 (1.0 g) and 0.5 M sodium hydroxide solution (11.76 ml) was stirred at 50 - 60 °C for 20 minutes. After cooling to ambient temperature, the pH of the solution was adjusted to about 2 with 1 M hydrochloric acid and the acidified solution was extracted twice with ether (20 ml). The extract was washed with brine (20 ml), dried over anhydrous sodium sulfate and concentrated under reduced pressure to give 1.0 g of the objective compound.
IR (neat, $cm^{-1}$) : 2929, 2861, 1718, 1459, 1251, 787
NMR ($CDCl_3$, δ): 3.88 - 3.38 (1H, m), 2.39 (2H, t), 2.06 -1.04 (12H, m), 0.89 (3H, t)

Example 6

Preparation of (S)-5-hydroxydecanoic acid L-lysine salt.

To a solution of the product obtained in Example 5 (100 mg) in ethanol (1 ml) was added a solution of L-lysine (63 mg) in water (1 ml), and the mixture was stirred at ambient temperature for 20 minutes. The resulting solution was concentrated under reduced pressure, and the residue was washed with ether (2 ml) to give 160 mg of the objective compound.
melting point: 170.0 °C (decomposition)
$[\alpha]_D^{25}$ = +4.7 ° (c = 1.10, methanol)

optical purity (HPLC): >98% e.e.
IR (KBr, $cm^{-1}$): 2931, 1617, 1583, 1561, 1507, 1407
NMR (DMSO-$d_6$, $\delta$): 3.74 (2H, t), 3.02 (2H, t), 2.34 -2.08 (2H, m), 2.08 - 1.10 (18H, m), 0.86 (3H, m)

Examples of the methods for producing pharmaceutical preparation comprising a compound of the present invention are described in the following. The present invention is not limited within the following examples.

Example A Glanul

Glanuls were prepared from the mixture of the following components by conventional methods.

| | |
|---|---|
| Sodium (S)-5-hydroxydecanoate | 450 g |
| Sucrose | 390 g |
| Cornstarch | 140 g |
| Hydroxypropilcellulose | 20 g |
| Total | 1,000 g |

Example B Capsule

An uniform mixture of 990 g of potassium (S)-5-hydroxydecanoate and 10 g of magnesium stearate was filled into No. 1 hard gelatin capsules to contain 250 mg in each capsule.

Example C Liquid preparation for oral administration

| | |
|---|---|
| Sodium (S)-5-hydroxydecanoate | 10 g |
| Liquid glucose | 25 g |
| Sucrose | 25 g |
| Preservative | a very small amount |
| Purified water | to make 100 ml |

Example D Lyophilized preparation for injection

Five grams of sodium (S)-5-hydroxydecanoate were dissolved in distilled water for injection. To the solution, further distilled water for injection was added to make 100 ml. The resulting aqueous solution was sterilized through a membrane filter, and the filterate was filled in glass vials in 2 ml portion and lyophilized. The vials were then sealed.

Example E Aqueous solution for injection

Ten grams of (S)-5-hydroxydecanoic acid L-lysine salt were dissolved in distilled water for injection. To the solution, further distilled water for injection was added to make 100 ml. The resulting aqueous solution was sterilized through a membrane filter, and the filterate was filled in ampules in 5 ml portions by conventional methods. The ampules were then sealed.

Example F Suppository

Twenty-five grams of sodium (S)-5-hydroxydecanoate, which had been ground in a mortar into fine particles, were mixed with 15 g of polyethylene glycol 1500 and 60 g of polyethylene glycol 4000. The mixture was made into suppositories weighting 2 g each, by a melting method.

A compounds of the present invention has high-potency of inhibitory effect on ATP regulated $K^+$ channels in cardiac cells and can be used for treatment of cardiovascular diseases such as therapeutic and/or preventive composition for arrhythmia and ischemic heart diseases, or as therapeutic composition for diabetes. Furthermore, a compound of the present invention has large margin for safety, and composition comprising a compound of the present invention as an effective component can be useful for drugs.

A compound of the present invention is an optically active one ((S)-isomer) of an already known racemate and is prepared for the first time. A compound of the present invention has higher pharmacological activity than a known racemate, so that, it is desired that both the therapeutically and/or preventively effective dose and the incidence of side-effects of the compound is low.

It is desired that (S)-δ-decalactone, starting compound of the present invention, can be prepared inexpensively and in industrial scale from easily purchased (R)-δ-decalactone by the method of the present invention.

## Claims

1. A compound selected from the group consisting of (S)-5-hydroxydecanoic acid, a pharmaceutically acceptable salt, a solvate and a solvate of said salt thereof.

2. A process for producing a compound selected from the group consisting of (S)-5-hydroxydecanoic acid, a pharmaceutically acceptable salt, a solvate and a solvate of said salt thereof, comprising at least one of following steps, a) (R)-decalactone is hydrolyzed with an alkali and the carboxyl group of the product is protected by esterification; b) the esterified compound is converted to the (S)-acyl derivative, comprising inversion of the configuration at 5-position by reacting with an organic carboxylic acid in the presence of an azodicarboxylic acid dialkyl ester and triphenylphosphine; c) the resulting (S)-acyl derivative is hydrolyzed of its ester groups and the resulting compound is cyclized in acidic condition to be (S)-δ-decalactone; and d) after hydrolysis of (S)-δ-decalactone with an alkali, free (S)-5-hydroxydecanoic acid can be prepared by adjusting the pH to 2-3 approximately with a mineral acid or an organic acid and the resulting free (S)-5-hydroxydecanoic acid can be treated with one equivalent of a base to produce a salt of (S)-5-hydroxydecanoic acid.

3. A process for producing a compound selected from the group consisting of (S)-5-hydroxydecanoic acid, a pharmaceutically acceptable salt, a solvate and a solvate of said salt thereof, comprising at least one of following steps, a) (R)-decalactone is hydrolyzed with an alkali and the carboxyl group of the product is protected by esterification; b) the esterified compound is converted to the (S)-acyl derivative, comprising inversion of the configuration at 5-position by reacting with an organic carboxylic acid in the presence of an azodicarboxylic acid dialkyl ester and triphenylphosphine; and c) the resulting (S)-acyl derivative is hydrolyzed of its ester groups with an alkali.

4. A pharmaceutical composition containing, as active ingredient, an effective amount of a compound selected from the group consisting of (S)-5-hydroxydecanoic acid, a pharmaceutically acceptable salt, a solvate and a solvate of said salt thereof, in admixture with pharmaceutically acceptable carriers.

5. A pharmaceutical composition claimed in claim 4 which composition is useful for treatment of cardiovascular diseases.

6. A pharmaceutical composition claimed in claim 4 which composition is useful for treatment of arrhythmia.

European Patent Office

# EUROPEAN SEARCH REPORT

Application Number

EP 90114356.0

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | CHEMICAL ABSTRACTS, vol. 110, no. 15, April 10, 1989, Columbus, Ohio, USA A. MOSANDL et al. "Stereo-isomeric flavorsubstances XXIII.delta-Lactone flavor compounds-structure and properties of the enantiomers" page 592, column 2, abstract-no. 133 860s & Z. Lebensm.-Unters. Forsch. 1988,187(1),40-4 -- | 1,2 | C 07 C 59/01<br>C 07 C 51/09<br>A 61 K 31/19 |
| A | CHEMICAL ABSTRACTS, vol. 100, no. 17, April 23, 1984, Columbus, Ohio, USA H. OHNISHI et al. " 5-Hydroxydecanoic acid for treatment of cardiovascular disorders" page 364, column 1, abstract-no. 144 984e & DE - A1 - 3 323 264 ---- | 1,4-6 | TECHNICAL FIELDS SEARCHED (Int. Cl.5)<br>C 07 C 59/00<br>C 07 C 51/00<br>A 61 K 31/00 |

The present search report has been drawn up for all claims

| Place of search | Date of completion of the search | Examiner |
|---|---|---|
| VIENNA | 11-10-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document